# EUROPEAN PATENT APPLICATION

(11) **EP 2 003 472 A1**
(43) Date of publication of application: **17.12.2008**
(21) Application number: 07108913.0
(22) Date of filing: 25.05.2007
(51) Int. Cl.: G01S 15/89

(54) **Ultrasound device with improved isotropy of the spatial resolution pattern**

(71) Applicant: Koninklijke Philips Electronics N.V., 5621 BA Eindhoven (NL)
(72) Inventor: The designation of the inventor has not yet been filed
(74) Representative: Pet, Robert Jacob

(57) **Abstract**

The invention relates to a method and an ultrasound probe for imaging a volume by at least receiving ultrasound signals from the volume. Generally, the point spread function is anisotropic, leading to a scan being made with an anisotropic spatial resolution pattern (point spread function with specific spatial orientation depending on the orientation of the ultrasound probe), which in turn results in the spatial resolution of an image obtained through use of the ultrasound probe to be anisotropic as well. According to the invention, the isotropy of the spatial resolution of an image is improved by making a plurality of scans of a single volume, with different scans being made with different spatial resolution patterns and with data obtained in the different scans being 3-D spatially compounded.

## Description

### FIELD OF THE INVENTION

The invention relates to an ultrasound device comprising at least one ultrasound transducer arranged for making a scan of at least a first part of a volume by at least receiving ultrasound signals from the volume.

The invention further relates to a system for imaging an interior of a volume comprising such an ultrasound device.

The invention further relates to a medical image acquisition system for imaging interior of a volume comprising such an ultrasound device.

The invention further relates to an ultrasound probe.

The invention further relates to a method for making a scan of a volume by sending ultrasound signals into the volume and receiving ultrasound signals from the volume as a result of sending ultrasound signals into the volume.

### BACKGROUND OF THE INVENTION

An embodiment of an ultrasound device of this kind is known from US patent 6,530,885 B1. In the aforementioned patent, an ultrasonic probe is moved to scan a volumetric region of a body. The region is scanned by sending ultrasound signals into a two-dimensional slice of the region and receiving ultrasound signals from the slice of the region. As the ultrasonic probe is moved successive slices of the region are scanned. Targets within the region that is scanned are interrogated from multiple look directions. Data obtained from multiple look directions are compounded to form two dimensionally spatially compounded image data. Data obtained from different slices is processed for display in a three-dimensional display format. It is a characteristic of the known device that the quality of a displayed image of the region scanned can be improved.

### SUMMARY OF THE INVENTION

It is an object of the invention to improve the quality of a displayed image of the region scanned. According to the invention of this object is realized in that the at least one ultrasound transducer further comprises:
- at least one further-scan ultrasound transducer arranged for making a further scan of at least a further part of the volume by at least receiving ultrasound signals from the volume, with the further part of the volume at least partially overlapping with the first part of the volume, with the further scan being made with a further-scan spatial resolution pattern, with the further-scan spatial resolution pattern being different from the first-scan spatial resolution pattern, and with the further-scan ultrasound transducer being arranged for producing further-scan image signals based on the ultrasound signals received by the further-scan ultrasound transducer;
   and in that the ultrasound device further comprises:
- a 3-D spatial compounding unit for spatially compounding the first scan and the further scan based on the first-scan image signals and the further-scan image signals.

The invention is based on the recognition that the spatial resolution of an ultrasound transducer is generally anisotropic. For an ultrasound transducer arranged for scanning a volume, for instance, the spatial resolution is generally different in the axial, azimuth, and elevation directions. This is because the size of the transducer aperture and the means of focusing the ultrasound beam generated by the transducer are likewise different in different directions. Typically, the transducer aperture is larger in the azimuth dimension as compared to the elevation dimension, and is dynamically focused using a 'beam former' that acts as an electronically variable lens. The transducer aperture is typically smaller in the elevation dimension as compared to the azimuth dimension and has a fixed focus using a physical cylindrical lens. The above results in a resolution in the azimuth dimension that is typically better than the resolution in the elevation dimension. Furthermore, even if the transducer aperture was equally sized and focused in azimuth and elevation, the axial resolution is typically better than the resolution in either the azimuth or elevation dimension.

The spatial resolution is characterized by the point spread function, the three-dimensional graphical interpretation of which is the resolution of an ultrasound device in three dimensions. In the example of the transducer described above, the graphical rendering of the point spread function would typically be that of a flattened rugby ball-like object, with the resolution in the elevation dimension defining the length of the point spread function, with the resolution in the azimuth dimension defining the width of the point spread function, and with the axial resolution defining the depth of the point spread function.

From the above it is clear that the point spread function relates to a spatial resolution pattern that has a certain shape, for instance, the rugby ball-like shape. However, the spatial resolution pattern also has a spatial orientation in a volume to be scanned depending on, for instance, the position and orientation of an ultrasound transducer relative to the volume to be scanned by the transducer. After all, the point spread function will move with the ultrasound transducer because the point spread function is defined by the properties of the transducer. Hence, it is possible to obtain a first scan of a volume with a first spatial resolution pattern and a further scan of at least a part of the same volume with a further spatial resolution pattern that is different from the first spatial resolution pattern. The spatial resolution patterns of the various scans will overlap, but will not be congruent. Consequently, combining at least two scans with different spatial resolution patterns through three-dimensional spatial compounding, for instance, by averaging, will result in a compounded spatial resolution pattern to which regions of the spatial resolution patterns that do not have overlap contribute less than regions that do overlap. By combining an increasing number of scans, with, for instance, each scan having been made with a different orientation of the ultrasound transducer used for the scans relative to the volume that is scanned, the resulting compounded spatial resolution pattern will become increasingly smaller and more isotropic. A more isotropic compounded spatial resolution pattern will result in an improved quality of a reconstructed image of the volume scanned. This is true because, through the invention and in contrast to the prior art, the volume is effectively scanned with a resolution that is increasingly similar in all dimensions, as an increasing number of scans having different spatial resolution patterns is compounded.

An embodiment of the device according to the invention, wherein the first-scan ultrasound transducer and the further-scan ultrasound transducer are comprised in a single, combined ultrasound probe, with the combined ultrasound probe being rotatable for providing the first-scan spatial resolution pattern and the further-scan spatial resolution pattern. This embodiment has the advantage that it enables improvement of the isotropy of the point spread function in a plane perpendicular to the rotation axis of the combined transducer as compared to the prior art, while using a single transducer.

A further embodiment of the device according to the invention, wherein the device comprises a plurality of combined ultrasound probes. This embodiment has the advantage that a plurality of combined ultrasound probes enables improvement of the isotropy of the point spread function not only in a plane perpendicular to the rotation axis of each combined ultrasound probe, but also in the direction of the rotation axis of each transducer. This can be achieved by compounding scans having been made by different combined ultrasound probes having rotation axes that are directionally independent. Hence, the spatial resolution patterns from different scans will not be congruent in the direction of each rotation axis.

A further embodiment of the device according to the invention, wherein the ultrasound device comprises a plurality of ultrasound transducers, with the plurality of ultrasound transducers being aligned using face normals of a regular polyhedron. This embodiment has the advantage that it adds additional symmetry to the totality of partially overlapping spatial resolution patterns as compared to the situation in which multiple ultrasound transducers are a less regularly distributed along the volume to be scanned than in the case of the polyhedron. As the overlapping regions of the various spatial resolution patterns contribute more to the combined point spread function that results after spatial compounding than non-overlapping regions, the additional symmetry will also increase the symmetry of the combined point spread function. In medical diagnostics, where a device according to this embodiment may be used for, for instance, imaging an interior of a female breast, an ultrasound transducer may be arranged such that its line of sight is substantially in a direction in which a particular region of interest of an object to be scanned, such as the armpit region near a female breast, is expected. Such an alignment has to do with the fact that tumors may be distributed inhomogeneously inside a breast, with a chance of finding a tumor in the armpit region being larger than the chance of finding a tumor more towards a patient's belly. Furthermore, such an alignment may fall within the scope of this embodiment if only by choosing the number of surfaces comprised in the regular polyhedron large enough. When aligning a plurality of ultrasound transducers using face normals of a regular polyhedron, the number of ultrasound transducers used may be less than the number of surfaces of the polyhedron. In such an instance, at least one face normal is not used for aligning an ultrasound transducer.

A further embodiment of the device according to the invention, wherein the first-scan ultrasound transducer and the further-scan ultrasound transducer are comprised in a wall structure bounding a space comprising the volume to be scanned. This embodiment has the advantage that a wall structure bounding a space comprising the volume to be scanned offers a convenient platform for arranging one or more ultrasound transducers for making scans having different spatial resolution patterns according to the invention. In a wall structure, different ultrasound transducers may, for instance, be orientated differently in accordance with the invention.

A further embodiment of the device according to the invention, wherein the wall structure substantially forms a cup. This embodiment has the advantage that a wall structure forming a cup defines a volume to be scanned, namely the inside of the cup and provides an easy means for arranging one or more ultrasound transducers for making scans of a substantial part of this volume, with different scans having different spatial resolution patterns according to the invention. A single combined ultrasound probe may, for instance, be comprised in the cup wall such that it faces the opening of the cup. From this position, the transducer can scan a substantial part of the volume of the cup with a first spatial resolution pattern, rotate through a predefined angle, and then make a further scan of at least a part of the volume scanned in the first scan with a further spatial resolution pattern that is different from the first spatial resolution pattern. A plurality of ultrasound transducers maybe arranged in different positions along the wall of the cup facing the volume to be scanned, with different transducers being orientated such that their spatial resolution patterns are different. Different ultrasound transducers may, for instance, be aligned using face normals of a regular polyhedron in accordance with the invention. A cup is especially suitable for the latter kind of alignment as it allows each transducer to be located at a position determined by the intersection of the surface of the cup facing the volume to be scanned with a line that is perpendicular to face normals of a regular polyhedron that is concentric with the cup. In the case of a cup having the shape of a half sphere, the centre of the cup is the centre of the sphere of which the cup forms one half.

A further embodiment of the device according to the invention, wherein the wall structure comprises compression surfaces. An object to be studied is accommodated between the compression surfaces after which are moved closer to each other, to apply some pressure to the object. This embodiment has the advantage that a wall structure according to the invention comprising compression surfaces enables scanning of an object in a fixed geometry, while, when the object is deformable, creating a region in which the object has a well-defined thickness. If the compression surfaces are two parallel plates this well-defined thickness is constant. For diffuse optical tomography a constant thickness has the advantage that light attenuation is limited in a range that is smaller than it would be if the object were not compressed.

The object of the invention is also realized with a system for imaging an interior of a volume, with the system comprising an ultrasound device according to any one of the previous embodiments and with the system further comprising a further-modality imaging unit. This embodiment has the advantage that the benefits from any one of the previous embodiments would also benefit a system comprising both an ultrasound device according to the invention and a further-modality imaging unit in addition to the ultrasound device. Interpretation of data obtained from the further mobility would benefit from the improved quality of the compounded ultrasound scan. Furthermore, the improved quality of the ultrasound data could be used in support of the further imaging modality, for instance by using ultrasound data in the reconstruction of an image obtained from the further imaging modality.

An embodiment of the system for imaging an interior of a volume according to the invention, wherein the further-modality imaging unit comprises a diffuse optical tomography imaging unit for imaging an interior of an optically turbid medium comprised within the volume, with the diffuse optical tomography imaging unit comprising:
- a light source for generating light to be coupled into the volume;
- a photodetector unit for detecting light emanating from the volume as a result of coupling light from the light source into the volume;
- an optical image reconstruction unit for reconstructing an image of an interior of the turbid medium based on light detected by the photodetector unit. A system for imaging an interior of a volume comprising both an ultrasound device and a diffuse optical tomography imaging unit would benefit from any one of the previous embodiments. If the system separately displays an ultrasound image and an optical image obtained through diffuse optical tomography interpretation of the latter would benefit from improvements in the former based on the invention. A similar benefit exists if the system displays a combination of an ultrasound image and an optical image obtained through diffuse optical tomography, with the combination being, for instance, an overlay of both images.

A further embodiment of the system for imaging an interior of a volume according to any one of the previous embodiments, wherein the system further comprises a multimodality image reconstruction unit for reconstructing a combined image of the volume based both on the further imaging modality and ultrasound signals received by at least one ultrasound transducer. This embodiment has the advantage that the quality of the combined image based both on the further imaging modality and on ultrasound signals received by at least one ultrasound transducer benefits from any one of the previous embodiments as the invention provides for better quality ultrasound input for the reconstruction of the combined image as compared to the prior art. With diffuse optical tomography the resolution of a reconstructed image is typically lower than the resolution of an ultrasound image. Furthermore, ultrasound is more suitable for imaging structures, whereas diffuse optical tomography allows the functional imaging of objects such as human tissues. Consequently, reconstruction of an image based on a scan that has been made using one imaging modality may benefit from using on a scan using the other imaging modality. Reconstructing an image based on both diffuse optical tomography and ultrasound would improve the resolution of the image as compared to the resolution of a diffuse optical tomography image (for instance a diffuse optical tomography image of which the resolution is enhanced using an ultrasound scan) and could also be used to combine the strengths of both imaging modalities (for instance an image in which both structural and functional features are displayed). Moreover, structural information relating to an object, such as human tissue, obtained from an ultrasound scan may be used in setting boundary conditions for the reconstruction of an optical image of the same object. This would improve the accuracy of the reconstruction of the optical image. Moreover still, using data obtained from an ultrasound scan in the reconstruction of an optical image allows a better estimate of the optical properties of the object imaged and hence of its composition. This is beneficial when imaging, for instance, an interior of a female breast.

A further embodiment of the system for imaging an interior of a volume according to any one of the previous embodiments, wherein the system is a medical image acquisition system. A medical image acquisition system for imaging an interior of a volume comprising both an ultrasound device and a diffuse optical tomography imaging unit would benefit from any one of the previous embodiments. If the system separately displays an ultrasound image and an optical image obtained through diffuse optical tomography interpretation of the latter would benefit from improvements in the first based on the invention.

The object of the invention is also a realized with an ultrasound probe comprising an ultrasound transducer arranged for making a first scan of a volume by at least receiving ultrasound signals from the volume, with the first scan being made with a first-scan spatial resolution pattern, with the ultrasound probe being further arranged for making a further scan of the volume by at least receiving ultrasound signals from the volume, with the further scan being made with a further-scan spatial resolution pattern, and with the further-scan spatial resolution pattern being different from the first-scan spatial resolution pattern. An ultrasound probe according to the invention has the advantage that it enables obtaining a number of scans of a volume, with different scans having different spatial resolution patterns. Through three-dimensional spatial compounding, different scans can be combined resulting in a combined point spread function that is smaller and more isotropic as compared to the point spread function of a volume scan obtained according to the prior art.

An embodiment of an ultrasound probe according to the invention, wherein the ultrasound transducer is rotatable for providing the first-scan spatial resolution pattern and the further-scan spatial resolution pattern. This embodiment has the advantage that it enables improvement of the isotropy of the point spread function in a plane perpendicular to the rotation axis of the combined transducer as compared to the prior art, while using a single transducer.

The object of the invention is also realized with a method for making an ultrasound scan of a volume, with the method comprising the following step:
- making a first scan of at least a first part of the volume by at least receiving ultrasound signals from the volume using a first-scan ultrasound transducer, with the first scan being made with a first-scan spatial resolution pattern and with the first-scan ultrasound transducer being arranged for producing first-scan image signals based on the ultrasound signals received by the first-scan ultrasound transducer, with the method further comprising the following steps:

- making a further scan of at least a further part of the volume by at least receiving ultrasound signals from the volume using at least one further-scan ultrasound transducer, with the further part of the volume at least partially overlapping with the first part of the volume, with the further scan being made with a further-scan spatial resolution pattern, with the further-scan spatial resolution pattern being different from the first-scan spatial resolution pattern, and with the further-scan ultrasound transducer being arranged for producing further-scan image signals based on the ultrasound signals received by the further-scan ultrasound transducer;
- 3-D spatial compounding of the first scan and the at least one further scan based on the first-scan image signals and the further-scan image signals using a 3-D spatial compounding unit. The method according to the invention has the advantage that it enables improvement of the spatial resolution and isotropy of the spatial resolution pattern of an image of a volume as compared to prior art.

An embodiment of the method for imaging interior of a volume according to the invention, wherein the method further comprises the following step:
- making a further modality scan of at least a third part of the volume, with the third part of the volume at least partially overlapping with the first and the further part of the volume and with the further modality scan being made with a further imaging modality. This embodiment has the advantage that interpretation of a scan obtained with the further imaging modality will benefit from the improved quality of the scan made using his ultrasound device according to the invention.

A further embodiment of the method for imaging an interior of a volume according to the invention, wherein the method further comprises the following step:
- reconstructing a combined image of an interior of the volume based both on the further imaging modality and ultrasound signals received by at least one ultrasound transducer using a multimodality image reconstruction unit. This embodiment has the advantage that one modality may be used in support of the other modality. For instance, data obtained from the ultrasound modality may be used in the reconstruction of an image based on data obtained from the other imaging modality.

A further embodiment of the method for imaging an interior of a volume according to the invention, wherein the further imaging modality comprises diffuse optical tomography. This embodiment would benefit from any one of the previous embodiments.

### BRIEF DESCRIPTION OF THE DRAWINGS

These and other aspects of the invention will be further elucidated and described with reference to the drawings, in which:
Fig. 1 schematically shows the point spread function produced by a typical 1D ultrasound transducer array;
Fig. 2 schematically shows an embodiment of a combined ultrasound probe for making scans with different spatial resolution patterns;
Fig. 3 shows spatial resolution patterns measured in the elevation-azimuth plane for different rotational positions of a combined ultrasound probe;
Fig. 4 schematically shows an embodiment of a cup comprising four ultrasound transducers aligned using face normals of a regular tetrahedron;
Fig. 5 schematically shows the overlapping spatial resolution patterns of four ultrasound transducers aligned using face normals of a regular tetrahedron;
Fig. 6 schematically shows the overlap of the fields of view of four ultrasound transducers aligned using face normals of a regular tetrahedron;
Fig. 7 schematically shows an embodiment of a system for imaging an interior of a volume comprising an ultrasound device according to the invention;
Fig. 8 schematically shows an embodiment of a medical image acquisition system comprising an ultrasound device according to the invention;
Fig. 9 schematically shows an embodiment of a method for making an ultrasound scan of a volume according to the invention and involving a further-modality scan.

### DETAILED DESCRIPTION OF EMBODIMENTS

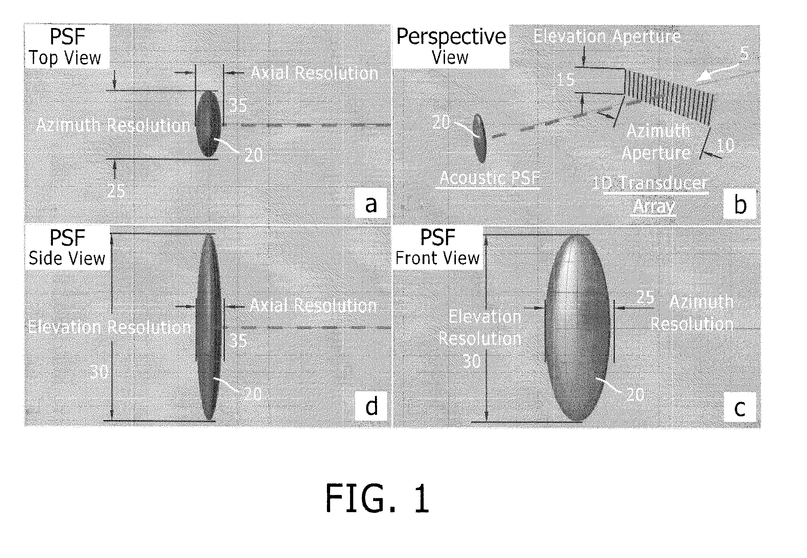

Fig. 1 schematically shows the point spread function produced by a typical 1D ultrasound transducer array. Fig. 1b schematically shows a typical 1D ultrasound transducer array as a series of vertical lines 5. The transducer aperture is larger in the azimuth dimension than in the elevation dimension. Typically, the transducer aperture in the azimuth dimension is dynamically focused using a 'beam former' that acts as an electronically variable lens. Similarly, in the elevation dimension, the transducer aperture typically has a fixed focus through use of a physical cylindrical lens. In fig. 1b the transducer aperture in the azimuth dimension is indicated by the number 10, whereas the transducer aperture in the elevation dimension is indicated by the number 15. The point spread function of the ultrasound transducer 5 is represented by the rugby ball-like object 20 in front of the transducer aperture. Figs. 1a, 1c, and 1d schematically show the point spread function 20 as seen from different directions, with fig. 1a showing a top view, with fig. 1c showing a front view, and with fig. 1d showing a side view. The differences in aperture size and focusing in the azimuth and elevation dimensions discussed above, typically result in the spatial resolution in the azimuth dimension 25 being better than the spatial resolution in the elevation dimension 30. However, even if the transducer aperture was equally sized and focused in the azimuth and elevation dimensions, the axial resolution 35 is typically better than the spatial resolution in either the azimuth or elevation dimension. The axial resolution 35 is independently determined by the transducer bandwidth and the resulting pulse length, regardless of aperture size. From fig. 1 the anisotropy of the point spread function of a typical ultrasound transducer array is clear. It is also clear that the point spread function being a characteristic of the ultrasound transducer moves with a transducer when the transducer itself is moved, for instance, for the scanning of a volume. Hence, it is possible to obtain different scans of a volume with different scans having been made with their respective point spread functions being oriented differently. Thus, the spatial resolution patterns being formed by the respective point spread functions in the various orientations will, in general, partially overlap but not be congruent.

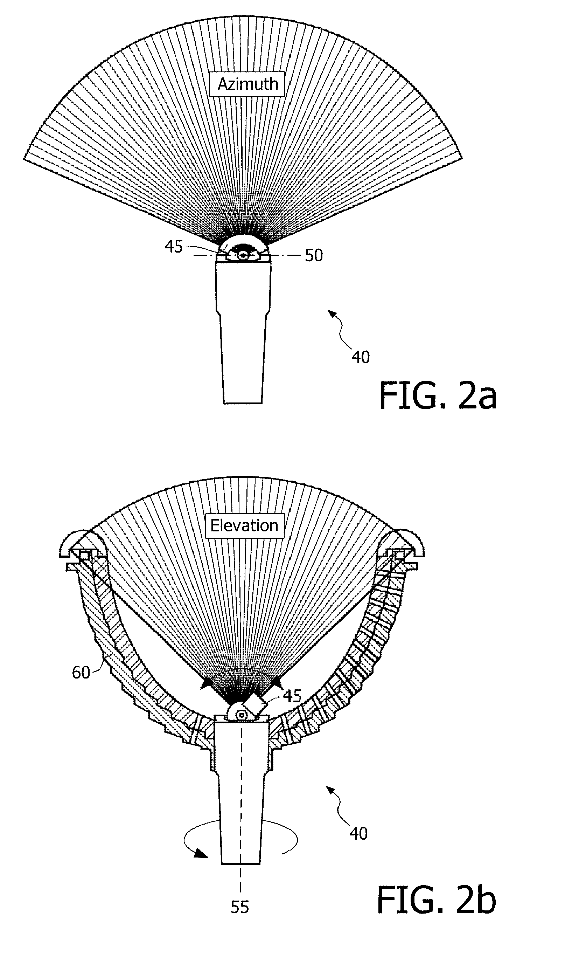

Fig. 2 schematically shows an embodiment of a combined ultrasound probe for making scans with different spatial resolution patterns. The combined ultrasound probe 40 comprises a scanner head 45 comprising, for instance, a curved linear array ultrasound transducer capable of making a sector scan in the azimuth dimension through electronic steering of the array. This is shown in fig. 2a in which the sector scan lies in the plane of the drawing. The combined ultrasound probe 40 further comprises an axis 50 around which the scanner head 45 can mechanically pivot. In this way, the ultrasound probe 40 can scan a volume through electronic steering in the azimuth dimension and mechanical steering in the elevation dimension, with the elevation dimension being perpendicular to the azimuth dimension. At each possible position relative to the pivot axis 50 a sector scan can be made in the azimuth dimension. The pivoting of the scanner head 45 around the pivoting axis 50 is schematically shown in fig. 2b that shows a side view of the combined ultrasound probe 40 shown in fig. 2a. The combined ultrasound probe 40 is arranged to be rotatable as a whole around an axis 55. At each angular position relative to the axis 55 the combined ultrasound probe 40 can scan a volume through electronic and mechanical steering of the scanner head 45 comprising the ultrasound transducer. The incremental angle A between each angular position relative to the axis 55 at which one out of a series of N scans is made may be determined by the formula A = 180°/N. Here, N can be extended to arbitrary positive integers. When, for example, N equals four, the incremental angle A equals 45°. This means that the combined ultrasound probe 40 will make a volume scan at 0°, 45°, 90° and 135° relative to the axis 55 and relative to an arbitrary starting point relative to this axis. As in fig. 2 the scanned volume is symmetric relative to the axis 55, scans at angular positions exceeding 180° are not required because these areas are already covered by scans made at angular positions below 180°. As explained in relation to fig. 1, the point spread function of an ultrasound transducer will move with the transducer when the transducer itself is moved. Hence, the combined ultrasound probe 40, being rotatable around the axis 55, allows different scans of a single volume to be obtained with different scans being made with different spatial resolution patterns, that is with the point spread functions of different scans having different spatial orientations. The spatial resolution patterns of scans having been made at different positions relative to the axis 55 will partially overlap. By combining different scans through 3-D spatial compounding, for instance, through averaging, the overlapping regions will contribute more to the compounded spatial resolution pattern than non-overlapping regions. Consequently, the compounded spatial resolution pattern that can be obtained through use of the combined ultrasound probe 40 will show an increased isotropy in a plane perpendicular to the axis 55 as compared to the spatial resolution pattern in scans according to the prior art. As in the direction of the axis 55 there is only one look angle, the isotropy of the compounded spatial resolution pattern in this direction will be substantially comparable to the isotropy that can be obtained in scans according to the prior art. In fig. 2b the combined ultrasound probe 40 is comprised in a cup 60 suitable for imaging an interior of a female breast. Through electronic and mechanical steering of the combined ultrasound probe 40, the transducer is capable of scanning a substantial part of the volume defined by the cup 60.

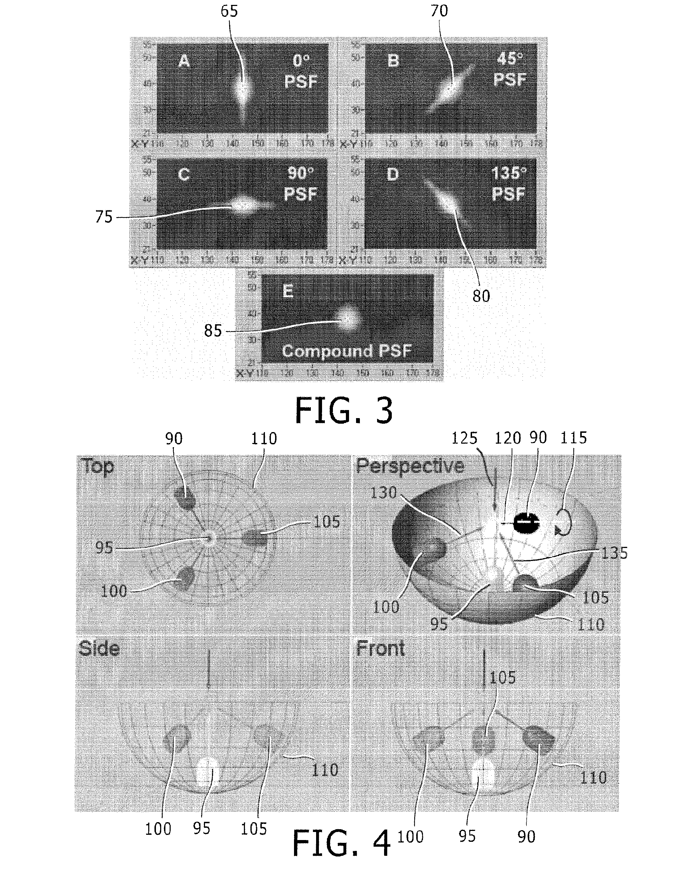

Fig. 3 shows spatial resolution patterns measured in the elevation-azimuth plane for different rotational positions of a combined ultrasound probe. The spatial resolution patterns 65, 70, 75, and 80 shown in figs. 3a-3d respectively have been measured at 0°, 45°, 90°, and 135° relative to an arbitrary starting point and relative to a rotational axis that is perpendicular to the elevation-azimuth plane (see also the discussion in relation to fig. 2). The various spatial resolution patterns shown are oriented differently relative to a single coordinate system because the point spread function moves with the ultrasound transducer as the transducer is rotated. Fig. 3e shows the compounded spatial resolution pattern 85 obtained by spatially compounding figs. 3a-3d. The central regions of the various spatial resolution patterns overlap, whereas the more outward lying regions do not. The central regions contribute more to the compounded spatial resolution pattern than the more outward lying regions resulting in a more isotropic spatial resolution pattern being obtained as compared to prior art.

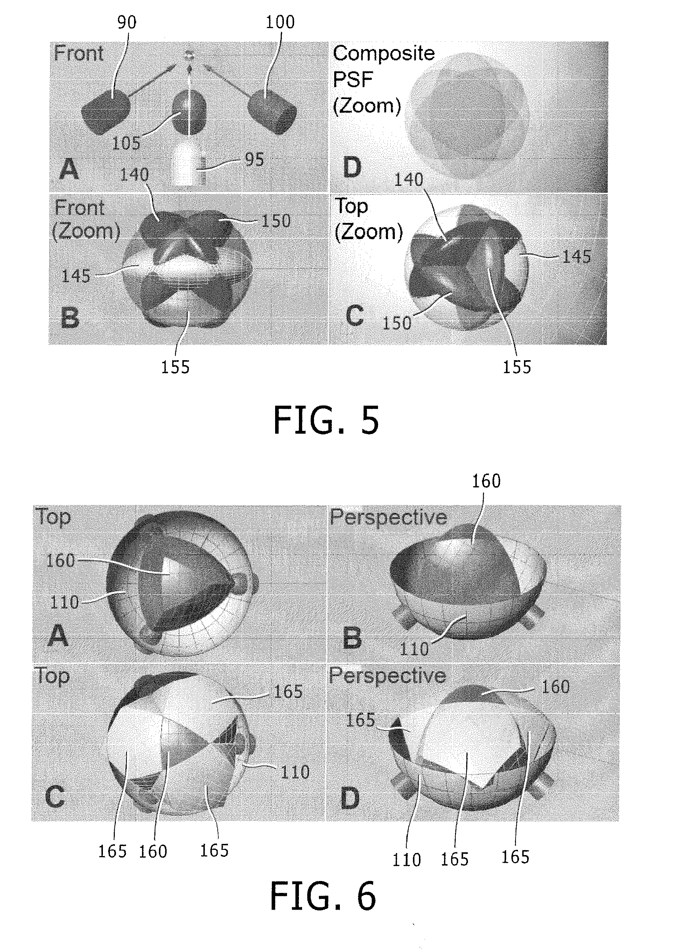

Fig. 4 schematically shows an embodiment of a cup comprising four ultrasound transducers aligned using face normals of a regular tetrahedron. Four ultrasound transducers numbered 90, 95, 100, and 105 respectively are mounted on a cup 110 comprising one half of a hollow sphere. Each of these four transducers is rotatable around a longitudinal axis 115 (shown only for transducer 90 for clarity). This principle was already discussed in relation to fig. 2 in which the combined ultrasound probe 40 was rotatable along an axis 55. Each transducer is arranged such that from it a compounded scan can be obtained with a compounded spatial resolution pattern that has improved isotropy in the elevation-azimuth plane as compared to prior art (see also the discussion in relation to fig. 3). Each transducer is mounted on the cup 110 at a location determined by the intersection of the surface of the hemispherical cup 110 that faces the volume to be scanned with a line defined by face normals of a regular polyhedron that is concentric with the imaginary sphere of which the cup 110 forms one half. For each of the ultrasound transducers 90, 95, 100, and 105 this line is indicated in fig. 4 by the arrows 120, 125, 130, and 135 respectively. These lines are mutually separated by angles of 120°. They also represent the central ultrasound beam emitted by each of the four transducers, which converge at the origin of the aforementioned imaginary sphere. Because of the planar symmetry of the point spread function associated with ultrasound transducer 95 relative to a plane perpendicular to arrow 125 (see also fig. 5), ultrasound transducer 95 can be comprised in the cup 110 with its general scanning direction facing the opening defined by the cup 110. Arranging a number of ultrasound transducers in accordance with face normals of a regular polyhedron introduces additional symmetry in the combined spatial resolution pattern obtained after compounding the scans made by the various ultrasound transducers as compared to a less regular arrangement of transducers.

Fig. 5 schematically shows the overlapping spatial resolution patterns of four ultrasound transducers aligned using face normals of a regular tetrahedron. An arrangement comprising four ultrasound transducers aligned using face normals of a regular tetrahedron was already discussed in relation to fig. 4. From each ultrasound transducer a compounded spatial resolution pattern is obtained that, starting from a rugby ball-like point spread function, looks more or less like a disk with rounded edges. This disk is obtained by combining several rugby ball-like point spread functions with different orientations. Fig. 5a essentially shows the arrangement already shown in fig. 4. In fig. 5b the compounded spatial resolution patterns obtained from ultrasound transducers 90, 95, 100, and 105, that is the discs, have been indicated by the numbers 140, 145, 150, and 155 respectively. When scans from all four transducers are additionally registered and compounded, for instance, by averaging, the resulting overall spatial resolution pattern will require additional symmetry and become substantially isotropic in three dimensions, as simulated in fig. 5d. The central region in this figure where all spatial resolution patterns overlap is the region that is reinforced by spatial compounding, and the fainter, outer regions are suppressed. The net result is a composite spatial resolution pattern that is highly symmetric and substantially isotropic in all three dimensions and whose size approaches the axial resolution of the original anisotropic point spread function (see also fig. 1). This results in a significant improvement in spatial resolution within at least a part of the volume that scanned where the images from all four transducers overlap. In this overlap region there is also a profound reduction of speckle noise, in this case on the other of the factor of √16, assuming that a total of four volume scans from each of the four transducers were compounded to form the composite space resolution pattern.

Fig. 6 schematically shows the overlap of the fields of view of four ultrasound transducers aligned using face normals of a regular tetrahedron. An arrangement comprising four ultrasound transducers aligned using face normals of a regular tetrahedron was already discussed in relation to fig. 4. Assuming that each transducer will scan a pyramid-like field of view having an apex measuring 130°x 130° in the azimuth and elevation dimensions, the scans from the four transducers will substantially overlap inside the cup 110. The maximum beneficial effect from the invention will occur in the central region 160 shown in figs. 6a and 6b. There is an additional region in which three out of four scans will overlap, shown as region 165 in figs. 6c and 6d. The compounded spatial resolution pattern in region 165 will be less symmetric and less isotropic than in the central region 160 and speckle will be reduced less because only 12 of the assumed 16 (see the discussion relation to fig. 5) scans will overlap. However, the effects will still be beneficial. Most of the remaining volume of the cup 110 will be covered by overlapping scans of two transducers, where the effect of the invention will be further reduced, but still worthwhile. It will be obvious to one skilled in the art that the same benefits of this embodiment could be achieved by using mechanical 3-D ultrasound transducers, which rotate or pivot a 1D linear, curvilinear, or phased array transducer to scan a volume or with a '2D' matrix linear, matrix curvilinear, or matrix phased array transducer. Also, the cup 110 does not have to be hemispherical, but could have an arbitrary shape. It will also be recognized that other numbers of transducers in different geometric arrangements are also possible according to this invention and would give beneficial effect similar to those described above. For example, instead of four transducers arranged in a tetrahedral pattern, three transducers could be arranged in a cubic pattern, where the transducers are lined with three face normals that are mutually separated by 90° angles. This arrangement allows spatial compounding in three dimensions with the minimum number of transducers required for such a task. Furthermore, this arrangement allows a compounded spatial resolution pattern to be obtained the size of which is typically substantially similar to that of the smallest dimension of the constituent spatial resolution patterns (see also fig. 5d showing a compounded spatial resolution pattern that is highly symmetric and substantially isotropic in three dimensions and whose size approaches the axial resolution of the constituent anisotropic point spread functions). Furthermore still, this arrangement would also produce a compounded spatial resolution pattern with symmetry and isotropy in three dimensions. Likewise, the number of transducers can be increased and they can be arranged in accordance with the patterns defined by face normals of regular polyhedrons and according to the method of the invention create a compounded space resolution pattern with symmetry and isotropy in three dimensions. Reducing the number of transducers to only two, which could be mounted, for instance, at right angles to each other, would provide improvement, but would not have the desirable three-dimensional symmetry and isotropy of the tetrahedral arrangement described above. This invention may optionally be combined with means for co-acquisition and/or post acquisition image registration and fusion of diffuse optical tomography and/or photoacoustic measurements within the cup. This invention may optionally be combined with means for co-acquisition and/or post acquisition image registration and fusion with MRI, CT, x-ray, or other imaging modalities. Finally, it will be obvious that this invention can be extended to form high-resolution three-dimensional ultrasound images of various human body parts, including a female breast, and extremities, small animals, or any animate or inanimate object as deemed useful.

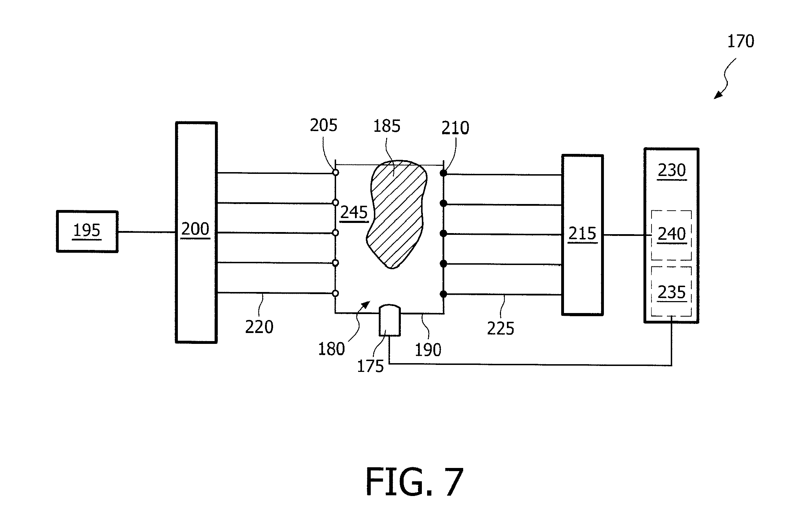

Fig. 7 schematically shows an embodiment of a system for imaging an interior of a volume comprising an ultrasound device according to the invention. The system 170 comprises an ultrasound transducer 175 arranged for making at least two volume scans, with different scans being made with different spatial resolution patterns according to the invention. The ultrasound transducer 175 may, for instance, be similar to ultrasound transducer 140 discussed in relation to fig. 2. In accordance with an embodiment of the invention the system 170 further comprises a receiving volume 180 for receiving an optically turbid medium 185. The ultrasound transducer 175 scans the receiving volume 180 by at least receiving ultrasound signals from the receiving volume 180. Ultrasound signals may be received, for instance, in response to sending ultrasound signals into the receiving volume 180 using the ultrasound transducer 175. However, another procedure from which received ultrasound signals might result is photoacoustic measurements. In that case sending ultrasound signals into the receiving volume 180 is not always required. The receiving volume 180 is limited by a cup-like wall 190. In fig. 7 the ultrasound transducer 175 is arranged such that its general scanning direction faces the opening of the cup. In this way the transducer 175 can scan a substantial part of the receiving volume 180 while reducing the reflection of ultrasound waves at the surface of the wall 190 that faces the receiving volume 180 as compared to a situation which the ultrasound transducer 175 would, for instance, be positioned halfway up one side of the wall 190 where it would face the opposite side of the wall 190. Alternatively, the system 170 may, for instance, comprise a plurality of ultrasound transducers 175 arranged in accordance using face normals of a regular polyhedron (see also the discussion in relation to figs. 4-6). After a turbid medium 185 is accommodated in the receiving volume 180, light from an irradiation light source 195 is coupled into the receiving volume 180 through use of the selection unit 200. The light from the light source 195 is chosen such that it can propagate through the turbid medium 185 without causing fluorescence in the turbid medium 185. In medical diagnostics, where the system 170 may be used as a medical system for imaging, for instance, an interior of a female breast, light having a wavelength within the range of, for instance, 400 nm to 1400 nm is suitable for this purpose. Alternatively, the light from the light source 195 may be chosen such that it can propagate through the turbid medium 185 and excite a fluorescent agent comprised in the turbid medium 185. The selection unit 200 is used to successively select an entrance position for light from a plurality of entrance positions for light 205. Light emanating from the receiving volume 180 as a result of coupling light from the irradiation light source 195 into the receiving volume 180 exits the receiving volume 180 using a plurality of exit positions for light 210. For the sake of clarity, the entrance positions for light 205 and the exit positions for light 210 have been positioned at opposite sides of the wall 190. In reality, however, both types of position may be spread around the receiving volume 180. Light emanating from the receiving volume 180 is detected through use of a photodetector unit 215. The plurality of entrance positions for light 205 are optically coupled to the selection unit 200 using light guides 220. The plurality of exit positions for light 210 is optically coupled to the photodetector unit 215 using light guides 225. Multimodality image reconstruction unit 230 is used to reconstruct an image of an interior of the turbid medium 185 based on either on light detected by the photodetector unit 215 or on ultrasound signals detected by the ultrasound transducer 175 or on both. The multimodality image reconstruction unit 230 comprises a 3-D spatial compounding unit 235 for combining scans made with different spatial resolution patterns. The multimodality image reconstruction unit 230 further comprises an optical image reconstruction unit 240 that is used to reconstruct an image of an interior of the turbid medium 185 based on light detected using the photodetector unit 215. Inside the receiving volume 180, the turbid medium 185 may be surrounded by a matching medium 245. The matching medium 245 has optical properties, such as an absorption coefficient, similar to those of the turbid medium 185. In this way, boundary effects stemming from coupling light from the irradiation light source 195 into and out of the turbid medium 185 are reduced and optical short-circuits around the turbid medium 185 prevented. An optical short-circuit arises if light that has traveled through the receiving volume 180 but outside the turbid medium 185 has been attenuated less than light that has traveled through the turbid medium 185. In that case, the former light may dwarf the latter light at the photodetector unit 215. Similarly, the turbid medium 185 may be surrounded in the receiving volume 180 by a matching medium 245 having acoustic properties, such as the speed of sound, similar to those of the turbid medium 185. In this way, boundary effects stemming from coupling ultrasound waves into and out of the turbid medium 185 are reduced. In fig. 7 the receiving volume 180 is bound by a wall 190. However, this need not always be the case. Another embodiment of a system for imaging an interior of a turbid medium is that of a handheld device that may, for instance, be pressed against a side of a turbid medium. In that case, the measurement volume is the volume occupied by the part of the turbid medium from which light is detected as a result of irradiating the turbid medium.

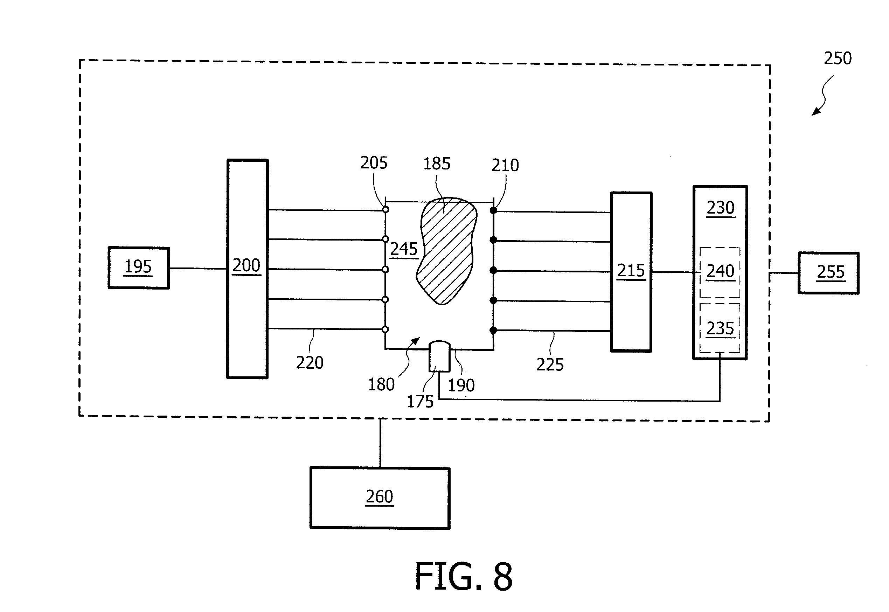

Fig. 8 schematically shows an embodiment of a medical image acquisition system comprising an ultrasound device according to the invention. The medical image acquisition system 250 comprises the elements of the system 170 shown in fig. 7 as indicated by the dashed square. Additionally, the medical image acquisition system 250 further comprises a screen 255 for displaying a reconstructed image of an interior of the turbid medium 185 and an operator interface 260, such as a keyboard, allowing an operator to interact with a the medical image acquisition system 250. Ultrasound images and diffuse optical tomography images may be displayed independently of one another. Alternatively, a combined image based on both ultrasound signals received by the ultrasound transducer 175 and on light detected by the photodetector unit 215 may be displayed.

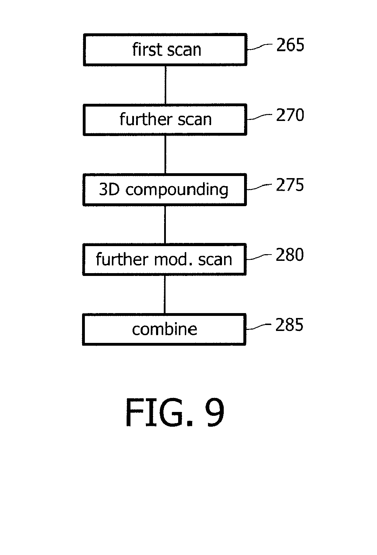

Fig. 9 schematically shows an embodiment of a method for making an ultrasound scan of a volume according to the invention and involving a further-modality scan. First in the method 263, in step 265, a first scan of at least a first part of the volume is made. This first scan is obtained by at least receiving ultrasound signals from the volume using a first-scan ultrasound transducer, with the first scan being made with a first-scan spatial resolution pattern and with the first-scan ultrasound transducer being arranged for producing first-scan image signals based on the ultrasound signals received by the first-scan ultrasound transducer. According to the invention the method further comprises step 270 in which a further scan is made of at least a further part of the volume by at least receiving ultrasound signals from the volume using at least one further-scan ultrasound transducer, with the further part of the volume at least partially overlapping with the first part of the volume, with the further scan being made with a further-scan spatial resolution pattern, with the further-scan spatial resolution pattern being different from the first-scan spatial resolution pattern, and with the further-scan ultrasound transducer being arranged for producing further-scan image signals based on the ultrasound signals received by the further-scan ultrasound transducer. The further scan may be obtained by rotating an ultrasound transducer as was, for instance, for instance described in relation to fig. 2. Another way of obtaining the further scan is to use a plurality of ultrasound transducers that are oriented differently with respect to the common volume that is scanned by all transducers. Still another way of obtaining the further scan is to use a plurality of rotatable ultrasound transducers. Through the rotation of each individual ultrasound transducer and acquiring a volume scan at a number of rotational positions, the isotropy of the spatial resolution pattern of the compounded scan that results from compounding the number of scans can be improved in a plane perpendicular to the rotation axis as compared to the isotropy of the spatial resolution pattern of each individual scan. By having multiple ultrasound transducers of which the rotational axes lie in spatially independent directions and combining scans obtained from these ultrasound transducers, a compounded spatial resolution pattern can be obtained with improved isotropy in the direction of the rotational axes. Next, in step 275, and still according to the invention, the first scan and the at least one further scan are 3-D spatially compounded to obtain the compounded scan, as discussed above, based on the first-scan image signals and the further-scan image signals using a 3-D spatial compounding unit. In step 280 a further modality scan is made of at least a third part of the volume, with the third part of the volume at least partially overlapping with the first and a further part of the volume and with a further modality scan being made with a further imaging modality. This further imaging modality may, for instance, be chosen from the group comprising: diffuse optical tomography imaging, MRI, CT, and x-ray. Interpretation of data obtained from the further imaging modality benefits from the availability of improved ultrasound data. A further imaging modality may further benefit from the invention if an image of an interior of the volume is reconstructed based on both the further imaging modality and ultrasound signals received by at least one ultrasound transducer using a multimodality image reconstruction unit according to the invention. Ultrasound data may, for instance, be used to enhance the resolution of a diffuse optical tomography image in which, because of the technology used, the resolution is typically limited. Moreover, diffuse optical tomography is suitable for the functional imaging of, for instance, human tissue. Diffuse optical tomography may, for instance, be used to determine the oxygenation in human tissue such as a female breast. Similarly, other imaging technologies, such as MRI, CT, and x-ray, also have their own particular strengths. Ultrasound technology, in its turn of, is more suitable to image structures than diffuse optical tomography. By basing the reconstruction of an image on both imaging modalities, the strengths of both technologies can be combined. In fig. 9 this is done in step 285.

It should be noted that the above-mentioned embodiments illustrate rather than limit the invention, and that those skilled in the art will be able to design many alternative embodiments without departing from the scope of the appended claims.
In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements, and by means of a suitably programmed computer. In the system claims enumerating several means, several of these means can be embodied by one and the same item of computer readable software or hardware. The mere fact that certain measures are recited in mutually different dependent claims does not indicate that a combination of these measures cannot be used to advantage.

## Claims

1. An ultrasound device (170) comprising at least one ultrasound transducer (175) arranged for making a scan of at least a first part of a volume (180) by at least receiving ultrasound signals from the volume (180), with the at least one ultrasound transducer (175) comprising:
- a first-scan ultrasound transducer arranged for making a first scan of at least a first part of the volume (180) with the first scan being made with a first-scan spatial resolution pattern, and with the first-scan ultrasound transducer being arranged for producing first-scan image signals based on the ultrasound signals received by the first-scan ultrasound transducer, with the at least one ultrasound transducer (175) further comprising:
- at least one further-scan ultrasound transducer arranged for making a further scan of at least a further part of the volume (180) by at least receiving ultrasound signals from the volume (180), with the further part of the volume (180) at least partially overlapping with the first part of the volume (180), with the further scan being made with a further-scan spatial resolution pattern, with the further-scan spatial resolution pattern being different from the first-scan spatial resolution pattern, and with the further-scan ultrasound transducer being arranged for producing further-scan image signals based on the ultrasound signals received by the further-scan ultrasound transducer;
and with the ultrasound device (170) further comprising:
- a 3-D spatial compounding unit (235) for spatially compounding the first scan and the further scan based on the first-scan image signals and the further-scan image signals.

2. An ultrasound device (170) as claimed in claim 1, wherein the first-scan ultrasound transducer and the further-scan ultrasound transducer are comprised in a single, combined ultrasound probe (40), with the combined ultrasound probe (40) being rotatable for providing the first-scan spatial resolution pattern and the further-scan spatial resolution pattern.

3. An ultrasound device (170) as claimed in claim 2, wherein the device comprises a plurality of combined ultrasound probes (40).

4. An ultrasound device (170) as claimed in claim 1 or claim 3, wherein the ultrasound device (170) comprises a plurality of ultrasound transducers, with the plurality of ultrasound and transducers being aligned using face normals of a regular polyhedron.

5. An ultrasound device (170) as claimed in claims 1-4, wherein the first-scan ultrasound transducer and the further-scan ultrasound transducer are comprised in a wall structure (190) bounding a space comprising the volume (180) to be scanned.

6. An ultrasound device (170) as claimed in claim 5, wherein the wall structure (190) substantially forms a cup (60).

7. An ultrasound device (170) as claimed in claim 5, wherein the wall structure (190) comprises compression surfaces.

8. A system (170) for imaging an interior of a volume (180), with the system (170) comprising an ultrasound device (170) according to any one of claims 1-7 and with the system (170) further comprising a further-modality imaging unit.

9. A system (170) for imaging an interior of a volume (180) as claimed in claim 8,
wherein the further-modality imaging unit comprises a diffuse optical tomography imaging unit for imaging an interior of an optically turbid medium (185) comprised within the volume (180), with the diffuse optical tomography imaging unit comprising:
- a light source (195) for generating light to be coupled into the volume (180);
- a photodetector unit (215) for detecting light emanating from the volume (180) as a result of coupling light from the light source (195) into the volume (180);
- an optical image reconstruction unit (240) for reconstructing an image of an interior of the turbid medium (185) based on light detected by the photodetector unit (215).

10. A system (170) for imaging an interior of a volume (180) as claimed in claim 8 or claim 9, wherein the system (170) further comprises a multimodality image reconstruction unit (230) for reconstructing a combined image of an interior of the turbid medium (185) based both on the further imaging modality and ultrasound signals received by at least one ultrasound transducer (175).

11. A system (170) as claimed in any one of claims 8-10, wherein the system (170) is a medical image acquisition system (250).

12. An ultrasound probe (40) comprising an ultrasound transducer (175) arranged for making a first scan of a volume (180) by at least receiving ultrasound signals from the volume (180), with the first scan being made with a first-scan spatial resolution pattern with the ultrasound probe (40) being further arranged for making a further scan of the volume (180) by at least receiving ultrasound signals from the volume (180), with the further scan being made with a further-scan spatial resolution pattern, and with the further-scan spatial resolution pattern being different from the first-scan spatial resolution pattern.

13. An ultrasound probe (40) as claimed in claim 12, wherein the ultrasound transducer (175) is rotatable for providing the first-scan spatial resolution pattern and the further-scan spatial resolution pattern.

14. A method (263) for making a scan of a volume (180), with the method (263) comprising the following step:
- making a first scan of at least a first part of the volume (180) by at least receiving ultrasound signals from the volume (180) using a first-scan ultrasound transducer, with the first scan being made with a first-scan spatial resolution pattern and with the first-scan ultrasound transducer being arranged for producing first-scan image signals based on the ultrasound signals received by the first-scan ultrasound transducer with the method (263) further comprising the following steps:
- making a further scan of at least a further part of the volume (180) by at least receiving ultrasound signals from the volume (180) using at least one further-scan ultrasound transducer, with the further part of the volume (180) at least partially overlapping with the first part of the volume (180), with the further scan being made with a further-scan spatial resolution pattern, with the further-scan spatial resolution pattern being different from the first-scan spatial resolution pattern, and with the further-scan ultrasound transducer being arranged for producing further-scan image signals based on the ultrasound signals received by the further-scan ultrasound transducer;
- 3-D spatial compounding of the first scan and the at least one further scan based on the first-scan image signals and the further-scan image signals using a 3-D spatial compounding unit (235).

15. A method (263) as claimed in claim 14, wherein the method (263) further comprises the following step:
- making a further modality scan of at least a third part of the volume (180), with the third part of the volume (180) at least partially overlapping with the first and the further part of the volume (180) and with the further modality scan being made with a further imaging modality.

16. A method (263) as claimed in claim 15, wherein the method (263) further comprises the following step:
- reconstructing a combined image of an interior of the volume (180) based both on the further imaging modality and ultrasound signals received by at least one ultrasound transducer (175) using a multimodality image reconstruction unit (230).

17. A method (263) as claimed in claims 14-16, wherein the further imaging modality comprises diffuse optical tomography.
